# EUROPEAN PATENT SPECIFICATION

(11) **EP 1 757 581 B1**
(45) Date of publication and mention of the grant of the patent: **01.10.2008**
(21) Application number: 06254363.2
(22) Date of filing: 18.08.2006
(51) Int. Cl.: C07C 409/16, C07C 407/00

(54) **Preparation of high purity fluorinated peroxides**
Herstellung hochreiner fluorierter Peroxide
Préparation de péroxides fluorés de haute pureté

(30) Priority: 24.08.2005 US 211386
(43) Date of publication of application: 28.02.2007
(73) Proprietor: Air Products and Chemicals, Inc., Allentown, PA 18195-1501 (US)
(72) Inventor: Syvret, Robert George, Allentown, PA 18103-5451 (US); Campion, Beth Ann, Allentown, PA 18104 (US); Cooper, Gregory Alan, Wernersville, PA 19565 (US)
(74) Representative: Muir, Benjamin M. J.

(56) References cited:
- US-A- 320 264
- US-A- 3 202 718
- US-A- 5 622 682
- DATABASE INTERNET University of Minnesota; 1 November 2004 (2004-11-01), STEVEN CLAAS: "Soda-Lime Vapor Trap" XP002408077

## Description

### BACKGROUND OF THE INVENTION

Fluorinated peroxides are powerful oxidizing agents and have value as free radical generators and etchants in the manufacture of electronic components. Perfluorodimethyl peroxide or sometimes called bis(trifluoromethyl)peroxide is an example of a common fluoroperoxide employed in these applications.

One of the many requirements imposed by the electronic industry is that of high purity. The resulting product must be substantially free of unreacted contaminants. Because of the highly reactive and hazardous nature of the reactants and byproducts formed in the synthesis, there are many safety issues and extreme care is required in synthesis and recovery of high purity product.

The following articles and patents are representative of the art with respect to fluorinated peroxides including the production of perfluorodimethyl peroxide and their recovery.

USP 3,100,803 and USP 3,230,264 (CIP of '264) disclose the synthesis of perfluorodimethyl peroxide by the reaction of equal molar amounts of carbonyl fluoride with trifluoromethyl hypofluorite in the presence of a metal fluoride catalyst. Reaction temperatures generally are in excess of 200 °C. Samples of products are collected by condensing in traps cooled by liquid oxygen. The condensate then is separated by distillation. In other procedures, carbon monoxide is reacted with fluorine in various ratios, thus generating carbonyl fluoride and trifluoromethyl hypofluorite *in situ.*

USP 3,202,718 discloses a method for the preparation of bis (trifluoromethyl) peroxide (BTMP) by reacting carbonyl fluoride (COF₂) with chlorine trifluoride (CIF₃) at temperatures ranging from 0 to 300 °C, preferably between 100 and 250 °C, in the presence of fluoride salts. Isolation and purification of BTMP is effected by passing the reaction product through a tube of granulated calcium chloride, scrubbing with water and dilute caustic to remove residual chlorine, hydrogen fluoride, and carbonyl fluoride, passing the scrubbed stream through a -80 °C trap to freeze out water and condense the bis(trifluoromethyl)peroxide, and then passing the liquid stream through a liquid nitrogen trap to prevent loss of bis(trifluoromethyl) peroxide. When tetrafluoromethane is present as a byproduct, it is removed as a last step by distillation.

Roberts, H.L., Preparation of Bis(trifluoromethyl) Peroxide and its Reduction with Hexafluoropropene, J. Chem. Soc. (1964) 4538 discloses a process for the preparation of BTMP through the reaction of COF₂ with CF₃OF (molar ratio of 1.47:1) at high-temperature and high-pressure (275 °C and 780 psig/37.3 kPa) in a nickel autoclave. The autoclave is cooled to room temperature and the products remaining in the autoclave are removed and the gases passed through liquid air traps. Then the condensate is distilled in a Podbielniak column. The recovered product then is reacted with hexafluoropropene and a product consisting of telomers CF₃O]C₃F₆]ₙ-OCF₃ where n ≥2 is formed.

Gard, G. L., et al, Reactions of Xenon with Certain Strong Oxidizing Agents, The University of Chicago Press, Chicago, 1963 (p 109-111 disclose various reactions of xenon with various oxidizing agents. In one example xenon is reacted with trifluoromethyl hypofluorite to produce xenon difluoride and BTMP. In the recovery step, the reaction product at 225 °C is cooled in a water bath, then cooled to -78 °C and the volatile product pumped away and through a liquid nitrogen trap. The volatile materials other than xenon are shown to be CF₃OF, and CF₃OOCF₃ and the material remaining in the tube is xenon difluoride.

US 4,499,024 discloses a process for the production of bisfluoroxydifluoromethane (BDM) by the reaction of carbon dioxide and fluorine in the presence of a cesium fluoride catalyst, The principal impurities are CF₃OF, CO₂, and CF₄ although trace amounts of CF₃OOCF₃ can be present. Separation to produce high purity product is accomplished by liquefaction and venting the gaseous impurities.

US 4,654,444 discloses a process for producing fluorine containing diacylperoxides by the reaction of an acyl halide, e.g., α,α-difluoro-β-*t*-butoxypropionyl chloride with sodium peroxide in water and extracting the product with a suitable solvent.

### BRIEF SUMMARY OF THE INVENTION

This invention is directed to an improvement in a process for producing high-purity fluorinated peroxides typically formed by the reaction of a carbonyl fluoride with a hypofluorite. The improvement resides in a simplified process for the synthesis and gas phase recovery of high purity fluorinated peroxide product and comprises the steps:
(a) reacting the carbonyl fluoride with the hypofluorite under conditions such that a gaseous reaction product comprised of the fluorinated peroxide and unreacted carbonyl fluoride which is essentially free of unreacted hypofluorite is formed;
   said conditions comprising a stoichiometric excess of carbonyl fluoride to hypofluorite.
(b) removing unreacted carbonyl fluoride and any byproducts from the gaseous reaction product under gas phase conditions thereby generating a gaseous product stream containing the fluorinated peroxide; and then,
(c) collecting the fluorinated peroxide from step (b) in gas phase.

Significant advantages can be achieved using the simplified gas phase recovery process and some of these include:
an ability to produce fluorinated peroxides of sufficiently high purity to be used directly for electronics applications;
an ability to eliminate the low temperature condensation steps and distillation steps associated with prior purification processes;
an ability to produce such high purity fluorinated peroxide without the need for a distillation step; and,
an ability to reduce the hazards associated with low temperature recovery processes employing condensation in producing high purity fluorinated peroxides;

### DETAILED DESCRIPTION OF THE INVENTION

This invention is directed to an improvement in a process for the production of high purity fluorinated peroxides formed by reacting a carbonyl fluoride with a hypofluorite. The process is generally comprised of two steps, the first step involving the synthesis of the fluorinated peroxide containing reaction product and the second step involving the gas phase recovery of the fluorinated peroxide from the reaction product.

The first step in the process is comprised of reacting a carbonyl fluoride with a hypofluorite such as trifluoromethyl hypofluorite to form the fluorinated peroxide. The hypofluorite may be prepared prior to contact with the carbonyl fluoride or formed, *in situ,* as for example by the reaction of carbon monoxide with fluorine in the presence of a catalyst, e.g. a fluoride salt. Likewise the carbonyl fluoride can be formed prior to reaction with the hypofluorite or *in situ* as for example by reacting carbon monoxide with fluorine. Depending upon the reaction scheme selected reactants may be preformed or formed *in situ* as one desires.

Examples of hypofluorites suited for reaction include the C₁₋₈ alkylhypofluorites and C₁₋₈ bisalkylhypofluorites such as trifluoromethyl hypofluorite, chlorodifluoromethyl hypofluorite, pentafluoroethyl hypofluorite, heptafluoropropyl hypofluorite, and bis(fluoroxy)difluoromethane.

Preferred routes to desired fluorinated peroxides are described by the equations: 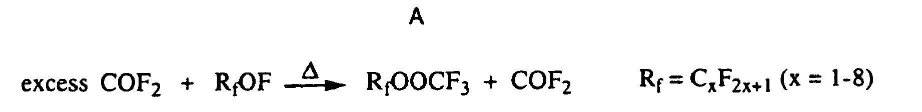 B excess COF₂ + R_{f}(OF)₂ → R_{f}(OOCF₃)₂ R_{f}=CₓF₂ₓ(x=1-8)

It has been found that one can reduce the hazards associated with the recovery of fluorinated peroxides including the simplification of the recovery process by initially controlling the reaction conditions associated with the synthesis. An initial objective in the synthesis of the fluorinated peroxide is one of effecting essentially complete conversion of the hypofluorite employed in the reaction. Reaction conditions are controlled to drive the equilibrium based reaction to completion leaving essentially no hypofluorite in the reaction product. It is preferred that the reaction product have less than 0.5% hypofluorite generally less than 0.1 % and preferably less than 0.05% by weight.

In a preferred embodiment, bis(trifluoromethyl) peroxide is produced by a process which comprises:
(a) reacting carbonyl fluoride with trifluoromethyl hypofluorite under conditions such that a gaseous reaction product comprised of bis(trifluoromethyl) peroxide, unreacted carbonyl fluoride and byproducts and having less than 0.5% trifluoromethyl hypofluorite is formed;
(b) removing unreacted carbonyl fluoride and by products from the gaseous reaction product under gas phase conditions thereby generating a gaseous product stream containing said bis(trifluoromethyl) peroxide; and then,
(c) collecting the bis(trifluoromethyl) peroxide from step (b) in gas phase.

A key factor leading to a reaction product essentially free of unreacted hypofluorite lies in the reaction stoichiometry. A stoichiometric excess of carbonyl fluoride to the hypofluorite is employed to drive the reaction as represented by the above equations to completion. Molar ratios of carbonyl fluoride to hypofluorite are generally from 1.1 to 3:1 and preferably from 1.2 to 1.7:1. Other byproducts commonly formed in the reaction in small to trace amount include carbon dioxide, hydrogen fluoride, fluorine, and adventitious water. Temperatures of from 150 to 350 °C and autogeneous pressures are typical. Reaction conditions vary depending on the hypofluorite used and the fluorinated peroxide being made.

It has been found that by conducting the reaction of carbonyl fluoride with the hypofluorite under conditions such that essentially no unreacted hypofluorite remains, one can implement a gas phase recovery process to achieve removal of the unreacted carbonyl fluoride and byproducts from the reaction product and recover the fluorinated peroxide without passing the reaction product through low temperature condensation traps. By maintaining gas phase conditions throughout recovery one avoids the hazards associated with highly reactive compounds in condensed form.

In the broadest sense recovery of the fluorinated peroxide from the gaseous reaction product can be achieved by passing the gaseous reaction product through a sorbent bed capable of removing the contaminating carbonyl fluoride, and any byproducts. Alkali exchanged 3 to 5 A zeolites and anhydrous soda-lime granules are exemplary of the adsorbents capable of removing such contaminants.

In the preferred recovery process the gaseous reaction product is initially scrubbed with an aqueous medium, typically an aqueous alkaline medium, in order to hydrolyze unreacted carbonyl fluoride, and neutralize any acidic compounds present in the gas stream. An alkaline solution having from 5 to 50% by weight of alkali, e.g., sodium hydroxide, potassium hydroxide, calcium hydroxide and so forth is a preferred scrubbing agent.

In the second part of the recovery process, the thus aqueous treated reaction product is passed through one or more absorptive beds to assist in the removal of unreacted carbonyl fluoride and byproduct carbon oxides, e.g., CO₂, and entrained H₂O. Representative adsorbents suited for removing contaminants from the fluorinated peroxide product include zeolites such as 3 and 4 A zeolites and soda-lime. Small pore zeolites should be used so as to avoid reaction with the fluorinated peroxide product. In the event that some carbon oxides remain after passage through the adsorbent bed, the product may be passed through a reactant such as soda-lime which is capable of reacting with the carbon oxides or another bed of adsorbent.

The following examples are provided to illustrate various embodiments of the invention.

### Example 1

### Preparation of Bis(trifluoromethyl)peroxide Mole Ratio 1.52:1

Reactants carbonyl fluoride, COF₂, and fluoroxytrifluoromethane, CF₃OF, were prepared in a flow system and collected in a clean and dry 600-cc Monel pressure reactor. A total of 500 mmol COF₂ and 328 mmol CF₃OF was collected representing a molar ratio of COF₂:CF₃OF of 1.52:1. The mixture of COF₂ and CF₃OF was heated under autogenous pressure to 274 °C over the course of 3.5 hours and then held at 274 °C for an additional 2 hours. After the specified time, the mixture was allowed to cool to ambient temperature. An infrared spectrum of the reactor contents indicated a mixture of COF₂ and bis(trifluoromethyl)peroxide (BTMP), CF₃OOCF₃, with no CF₃OF observed.

The gaseous contents of the reactor were passed through a purification train consisting of a column of H₂O followed by a bed of heat-activated 4Å molecular sieve and subsequently collected in a clean and dry collection cylinder at sub-ambient temperature. Infrared analysis of the product in the collection cylinder indicated high-purity CF₃OOCF₃ (BTMP) with no evidence of any CO₂, H₂O, COF₂, or CF₃OF present. The BTMP product weighed 33.3 g which represents a yield of 60%.

Using this reaction and recovery protocol one is able to avoid the hazards associated with the presence of CF₃OF in the reaction product and the separation hazards associated therewith.

### Example 2

### Preparation of Bis(trifluoromethyl)peroxide Mole Ratio of 1.25:1

In an experiment similar to that described in Example 1, 443 mmol of COF₂ and 354 mmol CF₃OF was collected representing a molar ratio of COF₂:CF₃OF of 1.25:1. The mixture of COF₂ and CF₃OF was heated under autogenous pressure to > 270 °C and then held between 270 °C and 280 °C for 3.5 hours. After the specified time, the mixture was allowed to cool to ambient temperature. An infrared spectrum of the reactor contents indicated a mixture of COF₂ and bis(trifluoromethyl)peroxide (BTMP), CF₃OOCF₃, with no CF₃OF observed.

The product of the reaction was purified and collected according to the method described in Example 1. Infrared analysis of the product in the collection cylinder indicated high-purity CF₃OOCF₃ (BTMP) with no evidence of any CO₂, H₂O, COF₂, or CF₃OF. The BTMP product weighed 36.9 g which represents a yield of 61%.

### Example 3

### Preparation of Bis(trifluoromethyl)peroxide Mole Ratio 1.99:1

Reactants carbonyl fluoride, COF₂, and fluoroxytrifluoromethane, CF₃OF, were distilled into a clean and dry 600-cc Monel pressure reactor. A total of 558 mmol COF₂ and 281 mmol CF₃OF was used representing a molar ratio of COF₂:CF₃OF of 1.99:1. The mixture of COF₂ and CF₃OF was heated under autogenous pressure to 275 °C over the course of 5.5 hours and then held at > 269 °C for an additional hour. After the specified time, the mixture was allowed to cool to ambient temperature. An infrared spectrum of the reactor contents indicated a mixture of CO₂, COF₂ and bis(trifluoromethyl)peroxide (BTMP), CF₃OOCF₃, with no CF₃OF observed.

The contents of the reactor were passed through a purification train consisting of a column of H₂O followed by a bed of heat-activated 4Å molecular sieves and the treated contents subsequently collected in a clean and dry collection cylinder at sub-ambient temperature. Infrared analysis of the product in the collection cylinder indicated a mixture of CF₃OOCF₃ (BTMP) and CO₂. Apparently the molecular sieves were not completely effective at removing CO₂. The contents of the collection cylinder were then passed through a column of soda-lime and collected in a clean dry cylinder. Analysis of the cylinder contents indicated high-purity CF₃OOCF₃ (BTMP) with no evidence of any CO₂, H₂O, COF₂, or CF₃OF.

This example shows that increasing the molar ratio of carbonyl fluoride to trifluoromethyl hypofluorite above 1.7, although effective for minimizing the level of trifluoromethyl hypofluorite in the reaction product, results in increased carbon oxide content, as well as increasing the level of unreacted carbonyl fluoride, and that increased load may place unnecessary burdens on the scrubbing and adsorbent systems in addition to the loss of reactant carbonyl fluoride.

### Example 4

### Purification of Crude Bis(trifluoromethyl)peroxide With Soda-Lime

A crude sample containing a mixture of BTMP and COF₂ was passed through a column of soda-lime and then collected in a clean and dry collection cylinder. Analysis of the cylinder contents indicated high-purity CF₃OOCF₃ (BTMP) with no evidence of any CO₂, H₂O, COF₂, or CF₃OF.

This example demonstrates that it is possible to employ a single adsorbent to remove substantially all of the contaminates from a reaction product containing the fluorinated peroxide.

### Example 5

### Purification of Crude Bis(trifluoromethyl)peroxide With Water and Soda-Lime

A crude sample containing a mixture of BTMP and COF₂ was passed through a column of H₂O and subsequently through a column of soda-lime and then collected in a clean and dry collection cylinder. Analysis of the cylinder contents indicated high-purity CF₃OOCF₃ (BTMP) with no evidence of any CO₂, H₂O, COF₂, or CF₃OF.

### Comparative Example 6

### Purification of Crude Bis(trifluoromethyl)peroxide With Soda-Lime

An attempt to purify a crude sample containing a mixture of BTMP and CF₃OF by passing the mixture through a column of soda-lime was not successful as the process generated oxygen as well as a tremendous amount of localized heat. That heat caused the subsequent decomposition of the remaining BTMP.

This example shows the unsuitability of the gas phase recovery of fluorinated peroxide product from a gaseous reaction product containing unreacted hypofluorite as is the case when COF₂ and CF₃OF are reacted in equal molar amounts. The scrubbing of the reaction product by contact with a column of water and removal of carbon oxides.

## Claims

1. A process for producing a high-purity fluorinated peroxide by reacting a carbonyl fluoride with a hypofluorite which comprises the steps:
(a) reacting said carbonyl fluoride with said hypofluorite under conditions such that a gaseous reaction product comprised of said fluorinated peroxide and unreacted carbonyl fluoride which is essentially free of unreacted hypofluorite is formed, said conditions comprising a stoichiometric excess of carbonyl fluoride to hypofluorite;
(b) removing unreacted carbonyl fluoride from the gaseous reaction product under gas phase conditions thereby generating a gaseous product stream containing said fluorinated peroxide; and then,
(c) collecting the fluorinated peroxide from step (b) in gas phase.

2. A process of Claim 1 wherein the reaction synthesis described in step (a) is represented by the formulas A and B: 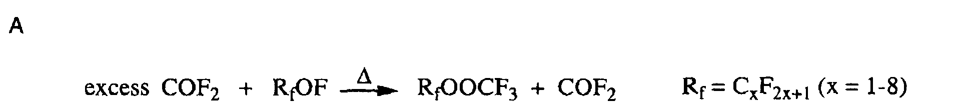
B excess COF₂ + R'_{f}(OF)₂ → R'_{f}(OOCF₃)₂ + COF₂ R'_{f} = CₓF₂ₓ (x = 1-8)

3. A process of Claim 1 or 2 wherein the mole ratio of carbonyl fluoride to hypofluorite is from 1.1 to 3:1.

4. A process of Claim 3 wherein said mole ratio is from 1.2 to 1.7:1.

5. A process of any one of the preceding claims wherein the gaseous reaction product is initially contacted with an aqueous medium under conditions for hydrolyzing unreacted carbonyl fluoride.

6. A process of Claim 5 wherein the gaseous reaction product is contacted with the aqueous medium under conditions for hydrolyzing carbonyl fluoride and forming an acidic compound.

7. A process of any one of the preceding claims wherein the gaseous reaction product stream, after the optional aqueous treatment, is passed through an absorbent to remove any unreacted carbonyl fluoride, byproducts and water.

8. A process of Claim 7 wherein the adsorbent is a zeolite molecular sieve.

9. A process of Claim 8 wherein the molecular sieve is a 3A or 4A molecular sieve.

10. A process of Claim 7 wherein the adsorbent is a molecular sieve or soda-lime.

11. A process of any one of the preceding claims wherein the hypofluorite is selected from C₁₋₈ alkylhypofluorites and C₁₋₈ bisalkylhypofluorites.

12. A process of Claim 11 wherein the hypofluorite is selected trifluoromethyl hypofluorite, chlorodifluoromethyl hypofluorite, pentafluoroethyl hypofluorite, heptafluoropropyl hypofluorite, and bis(fluoroxy)difluoromethane.

13. A process of Claim 12 wherein the hypofluorite is trifluoromethyl hypofluorite.

14. A process of any one of the preceding claims wherein unreacted trifluoromethyl hypofluorite in the reaction product is present in an amount of less than 0.5% by weight.

15. A process of Claim 14 wherein unreacted trifluoromethyl hypofluorite in the reaction product is present in an amount of less than 0.1 % by weight.

16. A process of Claim 15 wherein unreacted trifluoromethyl hypofluorite in the reaction product is present in an amount of less 0.05% by weight.

17. A process of any one of the preceding claims wherein the carbonyl fluoride and trifluoromethyl hypofluorite are preformed prior to reaction.

## Patentansprüche

1. Verfahren zur Herstellung eines fluorierten Peroxids hoher Reinheit durch Umsetzen eines Carbonylfluorids mit einem Hypofluorit, welches die folgenden Schritte umfasst:
(a) das Umsetzen des Carbonylfluorids mit dem Hypofluorit unter Bedingungen, so dass ein gasförmiges Reaktionsprodukt, welches das fluorierte Peroxid und nicht-umgesetztes Carbonylfluorid umfasst, das im Wesentlichen frei von nicht-umgesetztem Hypofluorit ist, gebildet wird, wobei die Bedingungen einen stöchiometrischen Überschuss von Carbonylfluorid zu Hypofluorit umfassen;
(b) das Entfernen von nicht-umgesetztem Carbonylfluorid von dem gasförmigen Reaktionsprodukt unter Gasphasenbedingungen, wobei ein gasförmiger Produktstrom, der das fluorierte Peroxid enthält, erzeugt wird; und dann
(c) das Sammeln des fluorierten Peroxids von Schritt (b) in Gasphase.

2. Verfahren nach Anspruch 1, wobei die in Schritt (a) beschriebene Reaktionssynthese durch die Formeln A und B dargestellt wird: 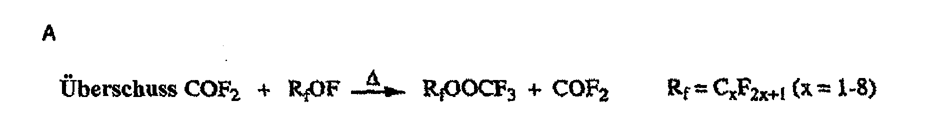
B Überschuss COF₂ + R'_{f}(OF)₂ → R'_{f}(OOCF₃)₂ + COF₂ R'_{f} = CₓF₂ₓ (x = 1.8)

3. Verfahren nach Anspruch 1 oder 2, wobei das Molverhältnis von Carbonylfluorid zu Hypofluorit 1,1 bis 3:1 beträgt.

4. Verfahren nach Anspruch 3, wobei das Molverhältnis 1,2 bis 1,7:1 beträgt.

5. Verfahren nach einem der vorhergehenden Ansprüche, wobei das gasförmige Reaktionsprodukt anfänglich mit einem wässrigen Medium unter Bedingungen zum Hydrolysieren von nicht-umgesetztem Carbonylfluorid in Kontakt gebracht wird.

6. Verfahren nach Anspruch 5, wobei das gasförmige Reaktionsprodukt mit dem wässrigen Medium unter Bedingungen zum Hydrolysieren von Carbonylfluorid und Bilden einer sauren Verbindung in Kontakt gebracht wird.

7. Verfahren nach einem der vorhergehenden Ansprüche, wobei der gasförmige Reaktionsproduktstrom nach der optionalen wässrigen Behandlung durch ein Absorbens geleitet wird, um jedwedes nicht-umgesetzte Carbonylfluorid, Nebenprodukte und Wasser zu entfernen.

8. Verfahren nach Anspruch 7, wobei das Adsorbens ein Zeolith-Molekularsieb ist.

9. Verfahren nach Anspruch 8, wobei das Molekularsieb ein 3 A- oder 4 A-Molekularsieb ist.

10. Verfahren nach Anspruch 7, wobei das Adsorbens ein Molekularsieb oder Natronkalk ist.

11. Verfahren nach einem der vorhergehenden Ansprüche, wobei das Hypofluorit aus C₁₋₈-Alkylhypofluoriten und C₁₋₈-Bisalkylhypofluoriten ausgewählt ist.

12. Verfahren nach Anspruch 11, wobei das Hypofluorit aus Trifluormethylhypofluorit, Chlordifluormethylhypofluorit, Pentafluorethylhypofluorit, Heptafluorpropylhypofluorit und Bis(fluoroxy)difluormethan ausgewählt ist.

13. Verfahren nach Anspruch 12, wobei das Hypofluorit Trifluormethylhypofluorit ist.

14. Verfahren nach einem der vorhergehenden Ansprüche, wobei nicht-umgesetztes Trifluormethylhypofluorit in dem Reaktionsprodukt in einer Menge von weniger als 0,5 Gew.-% vorhanden ist.

15. Verfahren nach Anspruch 14, wobei nicht-umgesetztes Trifluormethylhypofluorit in dem Reaktionsprodukt in einer Menge von weniger als 0,1 Gew.-% vorhanden ist.

16. Verfahren nach Anspruch 15, wobei nicht-umgesetztes Trifluormethylhypofluorit in dem Reaktionsprodukt in einer Menge von weniger als 0,05 Gew.-% vorhanden ist.

17. Verfahren nach einem der vorhergehenden Ansprüche, wobei das Carbonylfluorid und Trifluormethylhypofluorit vor der Umsetzung vorgebildet werden.

## Revendications

1. Procédé pour produire un peroxyde fluoré de pureté élevée par réaction d'un fluorure de carbonyle avec un hypofluorite qui comprend les étapes :
(a) réaction dudit fluorure de carbonyle avec ledit hypofluorite dans des conditions telles qu'un produit de réaction gazeux composé dudit peroxyde fluoré et de fluorure de carbonyle qui n'a pas réagit lequel est sensiblement exempt d'hypofluorite qui n'a pas réagit est formé, lesdites conditions comprenant un excès stoechiométrique de fluorure de carbonyle par rapport à l'hypofluorite ;
(b) élimination du fluorure de carbonyle qui n'a pas réagit du produit de réaction gazeux dans des conditions en phase gazeuse générant par ce moyen un courant de produit gazeux contenant ledit peroxyde fluoré ; et puis,
(c) collecte du peroxyde fluoré de l'étape (b) en phase gazeuse.

2. Procédé selon la revendication 1, dans lequel la réaction de synthèse décrite à l'étape (a) est représentée par les formules A et B :
COF₂ en excès 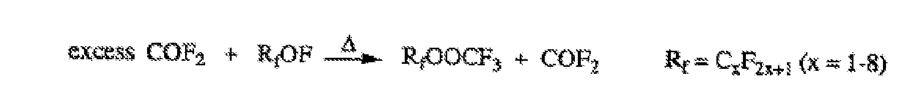
excess COF₂ + R'_{f}(OF)₂ → R'_{f}(OOCF₃)₂ + COF₂ R'_{f} = CₓF₂ₓ (x = 1-8)

3. Procédé selon la revendication 1 ou 2, dans lequel le rapport molaire du fluorure de carbonyle à l'hypofluorite est de 1,1 à 3:1.

4. Procédé selon la revendication 3, dans lequel ledit rapport molaire est de 1,2 à 1,7:1.

5. Procédé selon l'une quelconque des revendications précédentes, dans lequel le produit de réaction gazeux est d'abord mis en contact avec un milieu aqueux dans des conditions pour hydrolyser le fluorure de carbonyle qui n'a pas réagit.

6. Procédé selon la revendication 5, dans lequel le produit de réaction gazeux est mis en contact avec le milieu aqueux dans des conditions pour hydrolyser le fluorure de carbonyle et former un composé acide.

7. Procédé selon l'une quelconque des revendications précédentes, dans lequel le courant de produit réactionnel gazeux, après le traitement aqueux facultatif, est passé à travers un absorbant pour enlever tout le fluorure de carbonyle qui n'a pas réagit , les sous-produits et l'eau.

8. Procédé selon la revendication 7, dans lequel l'adsorbant est un tamis moléculaire zéolite.

9. Procédé selon la revendication 8, dans lequel le tamis moléculaire est un tamis moléculaire de 3A ou 4A.

10. Procédé selon la revendication 7, dans lequel l'adsorbant est un tamis moléculaire ou de la chaux sodée.

11. Procédé selon l'une quelconque des revendications précédentes, dans lequel l'hypofluorite est choisi parmi les alkylhypofluorites en C₁₋₈ et les bisalkylhypofluorites en C₁₋₈.

12. Procédé selon la revendication 11, dans lequel l'hypofluorite est choisi parmi l'hypofluorite de trifluorométhyle, l'hypofluorite de chlorodifluorométhyle, l'hypofluorite de pentafluoroéthyle, l'hypofluorite d'heptafluoropropyle, et le bis(fluoroxy)difluorométhane.

13. Procédé selon la revendication 12, dans lequel l'hypofluorite est l'hypofluorite de trifluorométhyle.

14. Procédé selon l'une quelconque des revendications précédentes, dans lequel l'hypofluorite de trifluorométhyle qui n'a pas réagit dans le produit réactionnel est présent dans une quantité inférieure à 0,5 % en poids.

15. Procédé selon la revendication 14, dans lequel l'hypofluorite de trifluorométhyle qui n'a pas réagit dans le produit réactionnel est présent dans une quantité inférieure à 0,1 % en poids.

16. Procédé selon la revendication 15, dans lequel l'hypofluorite de trifluorométhyle qui n'a pas réagit dans le produit réactionnel est présent dans une quantité inférieure à 0,05 % en poids.

17. Procédé selon l'une quelconque des revendications précédentes, dans lequel le fluorure de carbonyle et l'hypofluorite de trifluorométhyle sont préformés avant la réaction.
